# EUROPÄISCHE PATENTSCHRIFT

(11) **EP 3 054 913 B1**
(45) Veröffentlichungstag und Bekanntmachung des Hinweises auf die Patenterteilung: **28.03.2018**
(21) Anmeldenummer: 14799653.2
(22) Anmeldetag: 25.09.2014
(51) Int. Cl.: A61J 13/00

(54) **BRUSTWARZENSCHUTZVORRICHTUNG**
NIPPLE PROTECTOR
DISPOSITIF DE PROTECTION DU MAMELON

(30) Priorität: 07.10.2013 AT 3232013 U; 08.11.2013 AT 3662013 U
(43) Veröffentlichungstag der Anmeldung: 17.08.2016
(73) Patentinhaber: Pöltenstein, Markus, 1130 Wien (AT)
(72) Erfinder: Pöltenstein, Markus, 1130 Wien (AT)
(74) Vertreter: KLIMENT & HENHAPEL
(86) Internationale Anmeldenummer: PCT/AT2014/050220
(87) Internationale Veröffentlichungsnummer: WO 2015/051388

(56) Entgegenhaltungen:
- DE-A1- 10 033 891
- DE-C- 836 238
- US-A- 6 093 160
- US-A1- 2011 247 636

## Beschreibung

### GEBIET DER ERFINDUNG

Die vorliegende Erfindung betrifft eine Brustwarzenschutzvorrichtung sowie ein Set bestehend aus zwei solchen Brustwarzenschutzvorrichtungen.

Außerdem betrifft die vorliegende Erfindung die Verwendung einer erfindungsgemäßen Brustwarzenschutzvorrichtung, um ein Kleidungsstück von einer Brustwarze eines Benutzers / einer Benutzerin zu beabstanden.

### STAND DER TECHNIK

Insbesondere bei stillenden Frauen kann es durch das Stillen einerseits zu einer erhöhten Empfindsamkeit, andererseits zu einer Entzündung der Brustwarzen und gegebenenfalls des Warzenhofs kommen. In jedem Fall gilt es den Kontakt der Brustwarze bzw. des Warzenhofs mit Kleidungsteilen zu vermeiden, um einerseits eine Reizung, andererseits ein Verkleben mit der Brustwarze bzw. des/dem Warzenhof/s auszuschließen. Aus dem Stand der Technik sind hierzu im Wesentlichen schalenförmige Brustwarzenschutzvorrichtungen aus Silikon bekannt, die über die Brustwarzen samt Warzenhof gestülpt werden. Das Silikonmaterial sorgt für einen guten Sitz und Tragekomfort. Nachteilig hierbei ist jedoch, dass aufgrund der geringen Atmungsaktivität des Silikonmaterials Brustwarze und Warzenhof nässen. Einerseits kann dies an sich unangenehm sein, andererseits fördert das Nässen die Keimbildung, was wiederum für einen Heilungsprozess im Falle entzündeter Brustwarzen hinderlich ist.

Beispielsweise beschreibt die DE 836 238 C ein Brusthütchen zum Schutz und zur Heilbehandlung der weiblichen Brustwarze, wobei eine glockenförmig ausgebildete Hülle um die Brustwarze abzudecken und einzuhüllen und wobei das Brusthütchen mittels einer Klebemasse an der Haut befestigbar ist.

In der DE 100 33 891 A1 ist ein Brustwarzenschutz mit Innenaussparung für eine Brustwarze und einer die Brustwarze überdeckenden Außenfläche beschrieben.

Die US 2011/247636 A1 offenbart einen schützenden Heilbehelf für eine Brustwarze umfassend einen Grundkörper mit einer Kammer zur Aufnahme der Brustwarze, wobei die Kammer als durchgehende Öffnung im Grundkörper ausgebildet ist. Der Grundkörper besteht aus einem elastischen, saugfähigem Material, um Flüssigkeiten, wie Blut, aufsaugen zu können.

In der US 6,093,160 A ist eine Wundbehandlungsvorrichtung offenbart, die einen Anbringungsabschnitt, einen Übergangsabschnitt und einen Behandlungsabschnitt mit einer Wundabdeckung umfasst.

### AUFGABE DER ERFINDUNG

Es ist daher Aufgabe der vorliegenden Erfindung eine Brustwarzenschutzvorrichtung zur Verfügung zu stellen, die die oben genannten Nachteile vermeidet. Insbesondere soll ein Nässen von Brustwarze und/oder Warzenhof vermieden werden.

### DARSTELLUNG DER ERFINDUNG

Erfindungsgemäß wird die Aufgabe durch eine Brustwarzenschutzvorrichtung gelöst, die als eine Art Abstandhalter zwischen der Brustwarze bzw. dem Warzenhof einer Brust und einem Kleidungsstück, wie z.B. einem Büstenhalter, fungiert. Dabei wird die Brustwarze bzw. der Warzenhof in Richtung des Kleidungsstücks im Wesentlichen nicht abgedeckt, sodass eine ungehinderte Belüftung im Bereich der Brustwarze bzw. des Warzenhofs erzielt wird.

Diese Funktionalität wird durch einen ringförmigen Grundkörper der Brustwarzenschutzvorrichtung erreicht, der eine durchgehende Öffnung aufweist. Zum Schutz der Brustwarze bzw. des Warzenhofs wird die Brustwarzenschutzvorrichtung in eine Anwendungsposition gebracht, in welcher die Brustwarzenschutzvorrichtung zumindest abschnittsweise an der Brust aufliegt und Brustwarze bzw. Warzenhof in der Öffnung aufgenommen werden. In einer von der Brust weg weisenden Richtung bleiben Brustwarze und Warzenhof im Wesentlichen frei, was in diesem Bereich eine ungehinderte Luftzirkulation ermöglicht. Seitlich sind Brustwarze und Warzenhof von der Brustwarzenschutzvorrichtung umgeben. Wird nun ein Kleidungsstück von der Benutzerin / dem Benutzer übergezogen, werden der Kontakt und/oder ein Verkleben des Kleidungsstücks mit der Brustwarze bzw. dem Warzenhof unterbunden, indem das Kleidungsstück im Bereich der Brustwarze bzw. des Warzenhofs im Wesentlichen nur auf der Brustwarzenschutzvorrichtung anliegen kann.

Um den Komfort für die Benutzerin / den Benutzer zu erhöhen ist der Grundkörper aus einem elastischen Material gefertigt. Dies ermöglicht eine gewisse Anpassung bzw. ein gewisses Anschmiegen an die jeweilige Brust und vermeidet somit Druckstellen.

Ansprüche an die Hygiene werden erfüllt sowie der Komfort weiter gesteigert, indem über den Grundkörper ein Überzug aus Gewebe gezogen ist. Die Wahl des Gewebes kann dabei an die Vorgaben an Hygiene und Komfort angepasst werden. Beispielsweise ist es vorstellbar, ein besonders weiches Gewebematerial zu verwenden, um besonders empfindlicher Haut Rechnung zu tragen, oder ein besonders saugfähiges Gewebematerial zu verwenden, wenn die Brustwarze bzw. der Warzenhof bereits nässen sollten. Zudem kann nicht nur der Grundkörper, sondern insbesondere der Überzug entsprechend den hygienischen Anforderungen entsprechend gereinigt hergestellt werden, bevor er über den Grundkörper gezogen wird.

Daher ist in einer Brustwarzenschutzvorrichtung umfassend einen elastischen, im Wesentlichen ringförmigen Grundkörper mit einer durchgehenden Öffnung zur Aufnahme einer Brustwarze einer Brust und vorzugsweise eines die Brustwarze umgebenden Warzenhofs in einer Anwendungsposition der Brustwarzenschutzvorrichtung, erfindungsgemäß vorgesehen, dass die Brustwarzenschutzvorrichtung außerdem einen Überzug aus Gewebe umfasst, der über den Grundkörper gezogen ist, wobei die durchgehende Öffnung durch den Überzug nicht verdeckt ist. Wie gesagt wird zum Schutz der Brustwarze diese in der Öffnung derart vollständig aufgenommen, dass sie nicht über die Brustwarzenschutzvorrichtung hinaus absteht und die Brustwarzenschutzvorrichtung als Abstandhalter zum Kleidungsstück fungieren kann. Entsprechend ist es bei einer bevorzugten Ausführungsform der erfindungsgemäßen Brustwarzenschutzvorrichtung vorgesehen, dass die Brustwarzenschutzvorrichtung eine Höhe aufweist, die derart gewählt ist, dass in der Anwendungsposition die Brustwarze nicht über eine Vorderseite der Brustwarzenschutzvorrichtung hinaus ragt. Der Vorderseite gegenüberliegend weist die Brustwarzenschutzvorrichtung eine Rückseite auf, wobei in der Anwendungsposition die Rückseite zur Brust weist bzw. diese zumindest abschnittsweise berührt und die Vorderseite von der Brust weg weist.

Um eine an die Anatomie der Brustwarze bzw. des Warzenhofs besonders gut angepasste Form der Brustwarzenschutzvorrichtung zu erzielen, ist es bei einer bevorzugten Ausführungsform der erfindungsgemäßen Brustwarzenschutzvorrichtung vorgesehen, dass der Grundkörper einen kreisringförmigen Querschnitt aufweist, wobei die Querschnittsfläche normal auf eine Richtung steht, in welcher die Höhe gemessen ist. Parallel zu dieser Richtung können ebenfalls Querschnitte des Grundkörpers gebildet werden, die in diesem Fall auch rund, beispielsweise kreisförmig oder elliptisch sein können. Entsprechend variiert die konkrete Form des kreisringförmigen Querschnitts über die Höhe.

Selbstverständlich ist die Form des Grundkörpers nicht auf solche mit kreisringförmigem Querschnitt beschränkt. Insbesondere kann unterschiedlichsten Anatomien Rechnung getragen werden. Hierbei ist zwar stets eine durchgehende Öffnung vorhanden, doch kann eine äußere Umrandungslinie des Querschnitts des Grundkörpers von der Kreisform abweichen. Entsprechend ist es bei einer bevorzugten Ausführungsform der erfindungsgemäßen Brustwarzenschutzvorrichtung vorgesehen, dass der Grundkörper einen Querschnitt mit einer elliptischen äußeren Umrandungslinie aufweist, wobei die Querschnittsfläche normal auf eine Richtung steht, in welcher die Höhe gemessen ist. Analog ist es bei einer bevorzugten Ausführungsform der erfindungsgemäßen Brustwarzenschutzvorrichtung vorgesehen, dass der Grundkörper einen Querschnitt mit einer eirunden äußeren Umrandungslinie aufweist, wobei die Querschnittsfläche normal auf eine Richtung steht, in welcher die Höhe gemessen ist.

Eine Vielzahl von unterschiedlichen Querschnittsformen sowie unterschiedlichen Höhen kann realisiert werden, wenn der Grundkörper als Hohlzylinder ausgebildet wird. In diesem Fall variiert die konkrete Form des Querschnitts über die Höhe. Daher ist es bei einer bevorzugten Ausführungsform der erfindungsgemäßen Brustwarzenschutzvorrichtung vorgesehen, dass der Grundkörper die Form eines Hohlzylinders hat.

Im Falle der Ausführung des Grundkörpers als Hohlzylinder weist die Brustwarzenschutzvorrichtung im Allgemeinen eine Vorderseite und eine Rückseite auf, die beide im Wesentlichen als ebene Flächen ausgeführt sind, welche ebenen Flächen zueinander parallel sind. Um die Anpassung der Formgebung der Brustwarzenschutzvorrichtung an die Anatomie noch weiter zu verbessern, kann der Grundkörper zunächst so gestaltet werden, dass Vorderseite und Rückseite zwar ebene Flächen sind, welche ebenen Flächen aber zueinander nicht parallel sind. Darüberhinaus können Vorderseite und Rückseite auch als nicht ebene Flächen ausgeführt sein. Hierzu kann der Grundkörper eine Höhe aufweisen, die über dem Querschnitt des Grundkörpers variiert. Daraus ergibt sich, dass die Höhe der Brustwarzenschutzvorrichtung über dem Querschnitt des Grundkörpers variiert. Entsprechend ist es bei einer bevorzugten Ausführungsform der erfindungsgemäßen Brustwarzenschutzvorrichtung vorgesehen, dass die Höhe der Brustwarzenschutzvorrichtung über einem Querschnitt des Grundkörpers variiert, wobei die Querschnittsfläche normal auf eine Richtung steht, in welcher die Höhe gemessen ist.

Somit lassen sich unterschiedlichste Formen des Grundkörpers bzw. der Brustwarzenschutzvorrichtung vorsehen. Beispielsweise kann der Grundkörper bzw. die Brustwarzenschutzvorrichtung eine Keilform aufweisen. Entsprechend ist es bei einer für die Benutzerin / den Benutzer besonders angenehm zu tragenden Ausführungsform der erfindungsgemäßen Brustwarzenschutzvorrichtung erfindungsgemäß vorgesehen, dass die Brustwarzenschutzvorrichtung im Wesentlichen eine Keilform aufweist.

Um ein für die Benutzerin / den Benutzer besonders angenehmes elastisches Material des Grundkörpers zur Verfügung zu stellen und außerdem eine besonders kostengünstige Herstellung zu ermöglichen, ist es bei einer bevorzugten Ausführungsform der erfindungsgemäßen Brustwarzenschutzvorrichtung vorgesehen, dass der Grundkörper aus einem Schaumstoff, vorzugsweise Polyester-Schaumstoff besteht.

Um ein besonders angenehmes Tragegefühl einerseits sowie eine besonders einfache Herstellung andererseits zu erreichen, ist es bei einer bevorzugten Ausführungsform der erfindungsgemäßen Brustwarzenschutzvorrichtung vorgesehen, dass der Grundkörper einstückig ausgeführt ist.

Dies gestattet es, auf besonders einfache Weise hohe Stückzahlen herzustellen, indem der Grundkörper aus einem Schaumstoffteil, beispielsweise aus einer Schaumstoffbahn herausgestanzt wird. Entsprechend ist es bei einer bevorzugten Ausführungsform der erfindungsgemäßen Brustwarzenschutzvorrichtung vorgesehen, dass der Grundkörper ein Stanzteil ist.

Für die Befestigung des Überzugs am Grundkörper sind unterschiedliche Möglichkeiten vorstellbar, beispielsweise ein Vernähen, nachdem der Überzug über den Grundkörper gezogen ist. Eine besonders einfache und herstellungsgünstige Methode besteht darin, den Überzug als Schlauch mit einer gewissen Länge auszubilden, der zunächst von der Vorderseite (oder Rückseite) über die gesamte Höhe des Grundkörpers mit dessen gesamtem Durchmesser gestülpt wird. Hierauf wird der Überzug mit seiner verbleibenden Länge radial nach innen gestülpt und durch die Öffnung geführt und anschließend radial nach außen über die Rückseite (oder die Vorderseite) gestülpt. Anschließend wird der über die Vorderseite (Rückseite) abstehende Teil des Überzugs wieder radial nach innen über die Vorderseite (Rückseite) gestülpt und durch die Öffnung geführt und so fort, bis die gesamte Länge des Überzugs aufgebraucht ist. Entsprechend ist es bei einer bevorzugten Ausführungsform der erfindungsgemäßen Brustwarzenschutzvorrichtung vorgesehen, dass der Überzug schlauchförmig ausgebildet ist und über den Grundkörper mindestens einmal übergestülpt ist.

Um einen besonders hautfreundlichen Überzug zur Verfügung zu stellen, ist es bei einer bevorzugten Ausführungsform der erfindungsgemäßen Brustwarzenschutzvorrichtung vorgesehen, dass das Gewebe des Überzugs aus reiner Baumwolle besteht.

Insbesondere wenn eine oder beide Brustwarzen bzw. Warzenhöfe einer Benutzerin / eines Benutzers entzündet sind, ist es wichtig, dass nicht eine Brustwarzenschutzvorrichtung, die zwischenzeitlich auf einer Brust getragen wurde, in weiterer Folge auf der anderen Brust getragen wird. Dies hätte zur Folge, dass möglicherweise Keime aus dem Bereich der einen Brustwarze in den Bereich der anderen Brustwarze gelangen, was für einen Heilungsprozess hinderlich wäre. Um daher der Benutzerin / dem Benutzer eine einfache Möglichkeit zu geben sicherzustellen, dass nur eine bestimmte Brustwarzenschutzvorrichtung für eine Brust verwendet wird, ist es bei einer bevorzugten Ausführungsform der erfindungsgemäßen Brustwarzenschutzvorrichtung vorgesehen, dass die Brustwarzenschutzvorrichtung ein optisches Identifikationselement zur Identifikation der Brustwarzenschutzvorrichtung aufweist. Eine eindeutige Identifizierung und Zuordnung der jeweiligen Brustwarzenschutzvorrichtung zu einer bestimmten Brust ist somit leicht möglich.

Optische Identifikationselemente können beispielsweise durch reliefartige Strukturen an gewissen Stellen des Grundkörpers erzeugt werden, die sich durch den Überzug durchdrücken und daher sichtbar sind. Derartige Identifikationselemente sind darüberhinaus auch taktil erkennbar. Weiters sind z.B. Aufdrucke von Zeichen oder Mustern auf den Überzug denkbar. Ein weiteres Beispiel wäre die Farbgebung des Überzugs, wobei verschiedene Überzüge unterschiedliche Farben aufweisen können.

Schließlich ist es bei einer bevorzugten Ausführungsform der erfindungsgemäßen Brustwarzenschutzvorrichtung vorgesehen, dass das Identifikationselement als eine Naht des Überzugs ausgeführt ist, die sich optisch vom Rest des Überzugs abhebt.

Hierbei kann sich die Naht reliefartig vom Rest des Überzugs abheben. Alternativ oder zusätzlich kann sich die Naht farblich vom Rest des Überzugs abheben. Entsprechend ist es bei einer bevorzugten Ausführungsform der erfindungsgemäßen Brustwarzenschutzvorrichtung vorgesehen, dass sich die Naht farblich vom Rest des Überzugs abhebt.

Um sicherzustellen, dass der Benutzerin / dem Benutzer Brustwarzenschutzvorrichtungen zur Verfügung stehen, die sich auf die oben beschriebenen Arten jeder Brust eindeutig zuordnen lassen, ist erfindungsgemäß ein Set vorgesehen, umfassend zwei erfindungsgemäße Brustwarzenschutzvorrichtungen, wobei die beiden Brustwarzenschutzvorrichtungen mittels der Identifikationselemente optisch voneinander unterscheidbar sind.

Weiters ist es bei einer bevorzugten Ausführungsform der erfindungsgemäßen Brustwarzenschutzvorrichtung vorgesehen, dass im Grundkörper und/oder im Überzug ein Wirkstoff in Form einer Flüssigkeit oder Salbe aufgenommen ist, der in der Anwendungsposition der Brustwarzenschutzvorrichtung an die Brust abgebbar ist. Beispielsweise können der Grundkörper und/oder der Überzug mit einer einen Wirkstoff enthaltenden Flüssigkeit getränkt sein. Hierbei ist es auch möglich, einen medizinischen Wirkstoff vorzusehen, der einen Heilungsprozess der Brustwarze bzw. des Warzenhofs begünstigt. Grundsätzlich kann der Wirkstoff bei der Benutzerin / dem Benutzer aber auch einfach nur ein angenehmes Gefühl auslösen, z.B. durch einen kühlenden Effekt, sodass der Komfort gesteigert wird.

Generell kann die Brustwarzenschutzvorrichtung zur Erzielung eines Komfortgewinns verwendet werden, ohne dass dies in irgendeinem Zusammenhang mit einer medizinischen Indikation zu stehen braucht. Beispielsweise ist es im Sportbereich möglich, dass Sportler mit empfindlichen Brustwarzen bzw. Warzenhöfen erfindungsgemäße Brustwarzenvorrichtungen selbst verwenden und/oder von ihren Betreuern angelegt bekommen. Die Brustwarzenvorrichtungen können dabei mittels eines separaten elastischen Bandes am Körper gehalten werden oder durch ein, vorzugsweise stark konturiertes und straff anliegendes, übergezogenes Kleidungsstück, wie z.B. durch einen Büstenhalter. Daher ist erfindungsgemäß die Verwendung einer erfindungsgemäßen Brustwarzenschutzvorrichtung vorgesehen, um ein Kleidungsstück von einer Brustwarze einer Benutzerin / eines Benutzers zu beabstanden, zur Erhöhung des Komforts der Benutzerin / des Benutzers beim Tragen des Kleidungsstücks.

### KURZE BESCHREIBUNG DER FIGUREN

Die Erfindung wird nun anhand von Ausführungsbeispielen näher erläutert. Die Zeichnungen sind beispielhaft und sollen den Erfindungsgedanken zwar darlegen, ihn aber keinesfalls einengen oder gar abschließend wiedergeben.

Dabei zeigt:
- Fig. 1: eine Aufsicht auf einen Grundkörper einer erfindungsgemäßen Brustwarzenschutzvorrichtung
- Fig. 2: eine Seitenansicht einer erfindungsgemäßen Brustwarzenschutzvorrichtung
- Fig. 3: eine Schnittansicht einer erfindungsgemäßen Brustwarzenschutzvorrichtung in einer Anwendungsposition
- Fig. 4: eine Aufsicht auf einen Grundkörper, der einen Querschnitt mit einer elliptischen äußeren Umrandungslinie aufweist
- Fig. 5: eine Aufsicht auf einen Grundkörper, der einen Querschnitt mit einer eirunden äußeren Umrandungslinie aufweist
- Fig. 6: eine Seitenansicht einer keilförmigen Brustwarzenschutzvorrichtung
- Fig. 7: eine axonometrische Ansicht des Grundkörpers aus Fig. 4 mit Keilform
- Fig. 8: eine axonometrische Ansicht des Grundkörpers aus Fig. 5 mit Keilform

### WEGE ZUR AUSFÜHRUNG DER ERFINDUNG

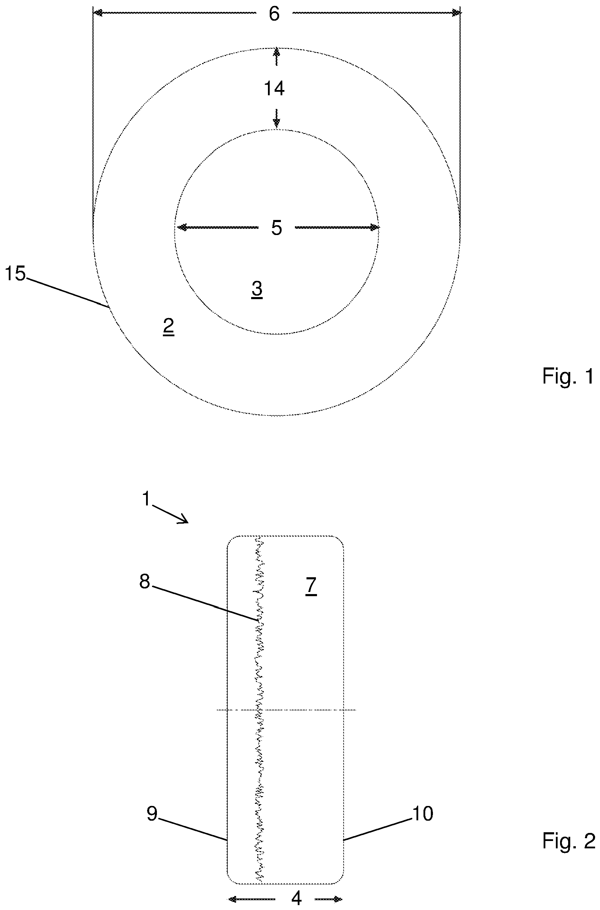

Fig. 1 zeigt einen Grundkörper 2 einer erfindungsgemäßen Brustwarzenschutzvorrichtung 1 in Aufsicht. Der Grundkörper 1 besteht aus elastischem Schaumstoff, vorzugsweise Polyester-Schaumstoff, und ist als Hohlzylinder mit einem kreisringförmigen Querschnitt ausgebildet. Entsprechend weist der Querschnitt eine äußere Umrandungslinie 15 auf, die kreisförmig ist, und es ergibt sich eine durchgehende Öffnung 3 im Grundkörper 1. Die Öffnung 3 weist einen Durchmesser 5 von beispielsweise 5 cm auf. Der Hohlzylinder hat eine Wandstärke 14 von beispielsweise 2 cm, sodass sich ein Durchmesser 6 des Grundkörpers 2 von beispielsweise insgesamt 9 cm ergibt. Im gezeigten Beispiel ist der Grundkörper 2 einstückig ausgeführt, wobei der Grundkörper 2 ein Stanzteil ist, das aus einem Schaumstoffstück, vorzugsweise einer Schaumstoffbahn herausgestanzt worden ist.

Die Brustwarzenschutzvorrichtung 1 weist außerdem einen Überzug 7 auf, mit dem der Grundkörper 2 überzogen ist, wie in der Seitenansicht der Fig. 2 erkennbar ist. Die durchgehende Öffnung 3 bleibt dabei erhalten bzw. wird nicht verdeckt. Der Überzug 7 kann beispielsweise als Schlauch ausgeführt sein, der über den Grundkörper 2 - vorzugsweise mehrfach - übergestülpt ist. Um ein angenehmes Tragegefühl zu erreichen, besteht der Überzug 7 aus einem Gewebe, vorzugsweise aus reiner Baumwolle.

Im gezeigten Ausführungsbeispiel der Fig. 2 weist der Überzug 7 eine Naht 8 auf, die sich optisch vom Rest des Überzugs 7 abhebt. Vorzugsweise wird dies durch eine geeignete Farbwahl der Naht 8 erreicht. Beispielsweise kann die Naht 8 rot, grün oder blau sein, wohingegen der restliche Überzug 7 in einer anderen Farbe gehalten ist, z.B. in Beige oder Weiß. Die Naht 8 fungiert auf diese Weise als Identifikationselement, das eine eindeutige Zuordnung der Brustwarzenschutzvorrichtung 1 ermöglicht.

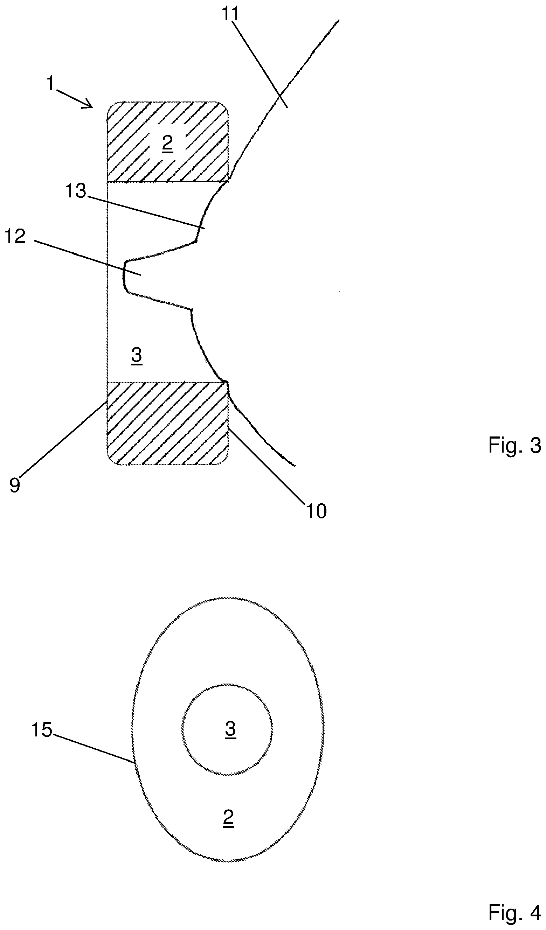

Dies ist insbesondere dann wichtig, wenn zwei Brustwarzenschutzvorrichtungen 1 - für jede Brust 11 (vgl. Fig. 3) eine -verwendet werden. In diesem Fall ermöglichen Nähte 8 in unterschiedlichen Farben die Unterscheidung der beiden Brustwarzenschutzvorrichtungen 1, sodass immer dieselbe Brustwarzenschutzvorrichtung 1 für dieselbe Brust 11 verwendet werden kann. Auf diese Weise wird z.B. verhindert, dass Verunreinigungen aus dem Bereich einer Brustwarze 12 der einen Brust 11 in den Bereich der Brustwarze 12 der anderen Brust 11 gelangen können. Entsprechend sind erfindungsgemäß Sets vorgesehen, die zwei Brustwarzenschutzvorrichtungen 1 umfassen, bei denen die Nähte 8 in unterschiedlichen Farben - z.B. in Rot und Blau - gehalten sind.

Gemäß Fig. 2 weist die Brustwarzenschutzvorrichtung 1 eine Vorderseite 9 und eine Rückseite 10 auf. Zwischen der Vorderseite 9 und der Rückseite 10 erstreckt sich die Brustwarzenschutzvorrichtung 1 mit einer Höhe 4 von beispielsweise 3 cm. Der Durchmesser 5 der Öffnung 3 und die Höhe 4 garantieren, dass in einer Anwendungsposition der Brustwarzenschutzvorrichtung 1 die Brustwarze 12 samt einem sie umgebenden Warzenhof 13 vollständig innerhalb der Öffnung 3 angeordnet werden kann, ohne dass die Brustwarze 12 über die Vorderseite 9 hinaus absteht bzw. ragt.

Dies ist aus Fig. 3 ersichtlich, die die Brustwarzenschutzvorrichtung 1 in der Anwendungsposition zeigt. Die Brustwarzenschutzvorrichtung 1 ist auf die Brust 11 aufgelegt und nimmt die Brustwarze 12 samt Warzenhof 13 vollständig innerhalb der Öffnung 3 auf. Dabei berührt die Brustwarzenschutzvorrichtung 1 mit der Rückseite 10 die Brust 1 zumindest abschnittsweise. Aufgrund der Elastizität des Grundkörpers 2 ergibt sich eine gewisse Anpassung der Form der Brustwarzenschutzvorrichtung 1 an die Form der jeweiligen Brust 11, um Druckstellen zu vermeiden und ein angenehmes Tragegefühl zu garantieren. Selbstverständlich können unterschiedliche Dimensionierungen der Durchmesser 5, 6 und der Höhe 4 vorgesehen werden, um auf unterschiedliche große Brustwarzen 12 bzw. Warzenhöfe 13 Rücksicht zu nehmen.

Die Brustwarzenschutzvorrichtung 1 kann in der gezeigten Anwendungsposition durch Anlegen eines Kleidungsstücks, beispielsweise eines Büstenhalters, gehalten werden und verhindert zuverlässig eine Kontaktierung und/oder ein Festkleben der Brustwarze 12 bzw. des Warzenhofs 13 mit dem Kleidungsstück. Da die Öffnung 3 durchgehend ist, werden Brustwarze 12 und Warzenhof 13 von der Vorderseite 9 nicht abgedeckt, sodass eine ungehinderte Luftzirkulation im Bereich von Brustwarze 12 und Warzenhof 13 erreicht wird.

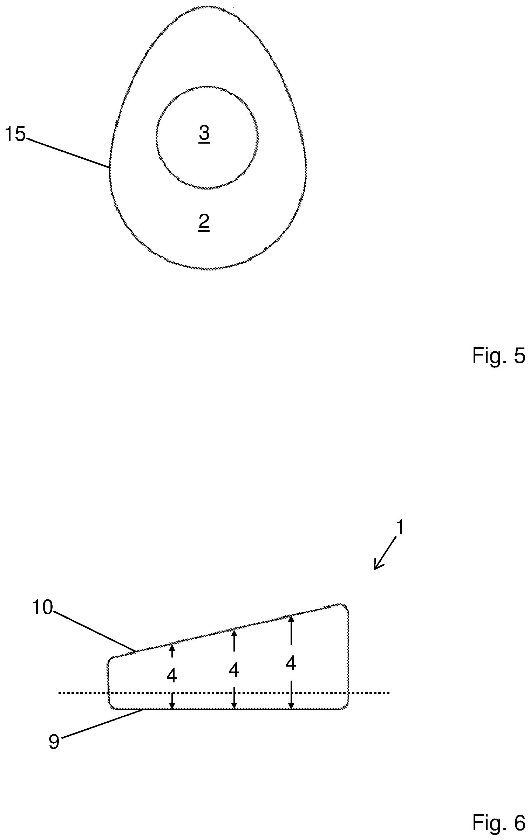

Um unterschiedlichsten Anatomien Rechnung zu tragen, kann der Grundkörper 2 einen Querschnitt aufweisen, der von der Kreisringform abweicht. Fig. 4 zeigt beispielsweise einen Grundkörper 2 mit einem Querschnitt, dessen äußere Umrandungslinie 15 elliptisch ist. Fig. 5 zeigt wiederum zeigt wiederum einen Grundkörper 2 mit einem Querschnitt, der eine eirunde äußere Umrandungslinie 15 aufweist.

Der Grundkörper 2 kann auch im Falle der Fig. 4 und der Fig. 5 als Hohlzylinder ausgeführt sein. Hierdurch ergeben sich für die Vorderseite 9 und die Rückseite 10 der Brustwarzenvorrichtung 1 im Wesentlichen ebene Flächen, die parallel zueinander liegen, vgl. Fig. 2 oder Fig. 3. Entsprechend ist die Höhe 4 der Brustwarzenschutzvorrichtung 1 über dem Querschnitt des Grundkörpers 2 konstant, vgl. Fig. 2.

Um die Anpassung der Form der Brustwarzenschutzvorrichtung 1 an unterschiedliche Anatomien weiter zu verbessern, kann die Brustwarzenschutzvorrichtung 1 auch derart gestaltet werden, dass deren Höhe 4 über dem Querschnitt des Grundkörpers 2 variiert. Hierdurch lässt sich beispielsweise eine Keilform der Brustwarzenschutzvorrichtung 1 erzeugen, wie in der Seitenansicht der Fig. 6 illustriert ist. Hierzu sei bemerkt, dass die Vorderseite 9 und Rückseite 10 in Fig. 6 natürlich auch umgekehrt angeordnet sein könnten.

Wie aus Fig. 6 ersichtlich ist, variiert die Höhe 4 der Brustwarzenschutzvorrichtung 1 über dem Querschnitt des Grundkörpers 2, wobei eine entsprechende Schnittebene, welche normal auf die Zeichenebene der Fig. 6 steht, durch die punktierte Linie angedeutet ist. Im Fall der Fig. 6 sind die Vorderseite 9 und die Rückseite 10 der Brustwarzenschutzvorrichtung 1 im Wesentlichen durch ebene Flächen ausgebildet. Es sei der Vollständigkeit halber jedoch festgehalten, dass bei einer über dem Querschnitt des Grundkörpers 2 variierenden Höhe 4 die Vorderseite 9 und Rückseite 10 der Brustwarzenschutzvorrichtung 1 im Allgemeinen nicht zwangsläufig ebene Flächen sein müssen.

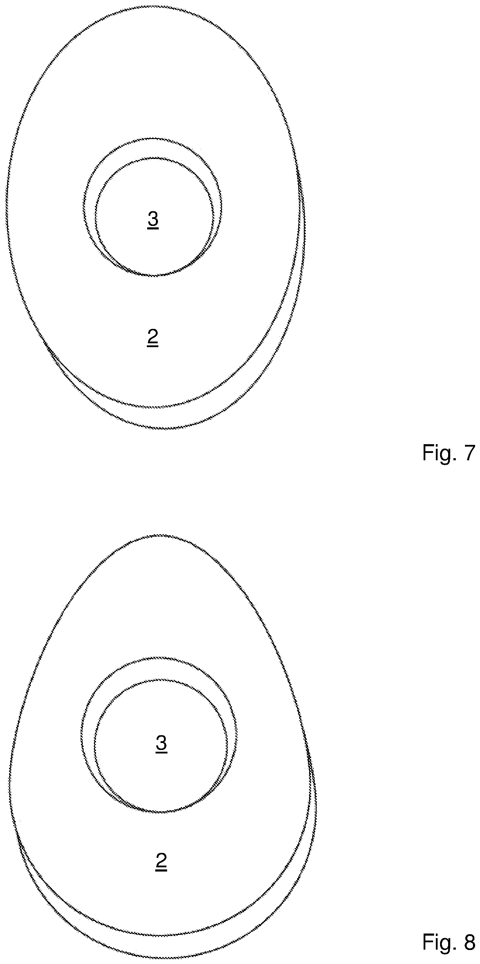

Die über dem Querschnitt des Grundkörpers 2 variierende Höhe 4 der Brustwarzenschutzvorrichtung 1 lässt sich am einfachsten dadurch realisieren, dass eine Höhe des Grundkörpers 2 über dessen Querschnitt variiert. Auf diese Weise lassen sich analog zu Fig. 6 keilförmige Grundkörper 2 erzeugen. Zur Illustration zeigt Fig. 7 eine axonometrische Ansicht eines keilförmigen Grundkörpers 2 für den in Fig. 4 illustrierten Fall eines Querschnitts mit elliptischer äußerer Umrandungslinie 15. Analog zeigt Fig. 8 eine axonometrische Ansicht eines keilförmigen Grundkörpers 2 für den in Fig. 5 illustrierten Fall eines Querschnitts mit eirunder äußerer Umrandungslinie 15.

Der Vollständigkeit halber sei bemerkt, dass eine Keilform der Brustwarzenschutzvorrichtung 1 beispielsweise auch bei einem hohlzylindrischen Grundkörper 2 ausgebildet werden könnte, indem z.B. der Überzug 7 entsprechend geformt ist (nicht dargestellt) oder indem zwischen Überzug 7 und Grundkörper 2 weitere Füllelemente angeordnet sind (nicht dargestellt).

### BEZUGSZEICHENLISTE

- 1: Brustwarzenschutzvorrichtung
- 2: Grundkörper
- 3: Öffnung
- 4: Höhe
- 5: Durchmesser der Öffnung
- 6: Durchmesser des Grundkörpers
- 7: Überzug
- 8: Naht
- 9: Vorderseite der Brustwarzenschutzvorrichtung
- 10: Rückseite der Brustwarzenschutzvorrichtung
- 11: Brust
- 12: Brustwarze
- 13: Warzenhof
- 14: Wandstärke
- 15: Äußere Umrandungslinie

## Patentansprüche

1. Brustwarzenschutzvorrichtung (1) umfassend einen elastischen, im Wesentlichen ringförmigen Grundkörper (2) mit einer durchgehenden Öffnung (3) zur Aufnahme einer Brustwarze (12) einer Brust (11) und vorzugsweise eines die Brustwarze (12) umgebenden Warzenhofs (13) in einer Anwendungsposition der Brustwarzenschutzvorrichtung (1), **dadurch gekennzeichnet, dass** die Brustwarzenschutzvorrichtung (1) außerdem einen Überzug (7) aus Gewebe umfasst, der über den Grundkörper (2) gezogen ist, wobei die durchgehende Öffnung (3) durch den Überzug (7) nicht verdeckt ist.

2. Brustwarzenschutzvorrichtung (1) nach Anspruch 1, **dadurch gekennzeichnet, dass** die Brustwarzenschutzvorrichtung (1) eine Höhe (4) aufweist, die derart gewählt ist, dass in der Anwendungsposition die Brustwarze (12) nicht über eine Vorderseite (9) der Brustwarzenschutzvorrichtung (1) hinaus ragt.

3. Brustwarzenschutzvorrichtung (1) nach Anspruch 2, **dadurch gekennzeichnet, dass** der Grundkörper (2) einen kreisringförmigen Querschnitt aufweist
oder dass der Grundkörper (2) einen Querschnitt mit einer elliptischen äußeren Umrandungslinie (15) aufweist oder dass der der Grundkörper (2) einen Querschnitt mit einer eirunden äußeren Umrandungslinie (15) aufweist, wobei die Querschnittsfläche jeweils normal auf eine Richtung steht, in welcher die Höhe (4) gemessen ist.

4. Brustwarzenschutzvorrichtung (1) nach einem der Ansprüche 1 bis 3, **dadurch gekennzeichnet, dass** der Grundkörper (2) die Form eines Hohlzylinders hat.

5. Brustwarzenschutzvorrichtung (1) nach einem der Ansprüche 2 bis 4, **dadurch gekennzeichnet, dass** die Höhe (4) der Brustwarzenschutzvorrichtung (1) über einem Querschnitt des Grundkörpers (2) variiert, wobei die Querschnittsfläche normal auf eine Richtung steht, in welcher die Höhe (4) gemessen ist.

6. Brustwarzenschutzvorrichtung (1) nach Anspruch 5, **dadurch gekennzeichnet, dass** die Brustwarzenschutzvorrichtung (1) im Wesentlichen eine Keilform aufweist.

7. Brustwarzenschutzvorrichtung (1) nach einem der Ansprüche 1 bis 6, **dadurch gekennzeichnet, dass** der Grundkörper (2) aus einem Schaumstoff, vorzugsweise Polyester-Schaumstoff besteht.

8. Brustwarzenschutzvorrichtung (1) nach einem der Ansprüche 1 bis 7, **dadurch gekennzeichnet, dass** der Grundkörper (2) einstückig, vorzugsweise als Stanzteil, ausgeführt ist.

9. Brustwarzenschutzvorrichtung (1) nach einem der Ansprüche 1 bis 8, **dadurch gekennzeichnet, dass** der Überzug (7) schlauchförmig ausgebildet ist und über den Grundkörper (2) mindestens einmal übergestülpt ist.

10. Brustwarzenschutzvorrichtung (1) nach einem der Ansprüche 1 bis 9, **dadurch gekennzeichnet, dass** das Gewebe des Überzugs (7) aus reiner Baumwolle besteht.

11. Brustwarzenschutzvorrichtung (1) nach einem der Ansprüche 1 bis 10, **dadurch gekennzeichnet, dass** die Brustwarzenschutzvorrichtung (1) ein optisches Identifikationselement (8) zur Identifikation der Brustwarzenschutzvorrichtung (1) aufweist.

12. Brustwarzenschutzvorrichtung nach Anspruch 11, **dadurch gekennzeichnet, dass** das Identifikationselement als eine Naht (8) des Überzugs (7) ausgeführt ist, die sich optisch vom Rest des Überzugs (7) abhebt.

13. Brustwarzenschutzvorrichtung (1) nach Anspruch 12, **dadurch gekennzeichnet, dass** sich die Naht (8) farblich vom Rest des Überzugs (7) abhebt.

14. Brustwarzenschutzvorrichtung (1) nach einem der Ansprüche 1 bis 13, **dadurch gekennzeichnet, dass** im Grundkörper (2) und/oder im Überzug (7) ein Wirkstoff in Form einer Flüssigkeit oder Salbe aufgenommen ist, der in der Anwendungsposition der Brustwarzenschutzvorrichtung (1) an die Brust (11) abgebbar ist.

15. Set umfassend zwei Brustwarzenschutzvorrichtungen (1) nach einem der Ansprüche 11 bis 14, wobei die beiden Brustwarzenschutzvorrichtungen (1) mittels der Identifikationselemente (7) optisch voneinander unterscheidbar sind.

## Claims

1. Nipple protector (1) comprising an elastic substantially annular base (2) having a continuous opening (3) for accommodating, in a use position of the nipple protector (1), a nipple (12) of a breast (11) and preferably an areola (13) surrounding the nipple (12), **characterized in that** the nipple protector (1) additionally comprises a fabric cover (7) which is pulled over the base (2), wherein the continuous opening (3) is not covered by the cover (7).

2. The nipple protector (1) according to claim 1, **characterized in that** the nipple protector (1) has a height (4) which is selected in such a manner that in the use position the nipple (12) does not project beyond a front side (9) of the breast protector (1).

3. The nipple protector (1) according to claim 2, **characterized in that** the base (2) has an annular cross-section or that the base (2) has a cross-section having an elliptical outer border line (15) or that the base (2) has a cross-section having an oval outer border line (15), wherein the cross-sectional surface is in each case normal to a direction in which the height (4) is measured.

4. The nipple protector (1) according to one of claims 1 to 3, **characterized in that** the base (2) has the form of a hollow cylinder.

5. The nipple protector (1) according to one of claims 2 to 4, **characterized in that** the height (4) of the nipple protector (1) varies over a cross-section of the base (2), wherein the cross-sectional surface is normal to a direction in which the height (4) is measured.

6. The nipple protector (1) according to claim 5, **characterized in that** the nipple protector (1) has a substantially wedge shape.

7. The nipple protector (1) according to one of claims 1 to 6, **characterized in that** the base (2) consists of foam material, preferably polyester foam material.

8. The nipple protector (1) according to one of claims 1 to 7, **characterized in that** the base (2) is designed in one piece, preferably as a punched part.

9. The nipple protector (1) according to one of claims 1 to 8, **characterized in that** the cover (7) is configured to be tubular and is pulled at least once over the base (2).

10. The nipple protector (1) according to one of claims 1 to 9, **characterized in that** the fabric of the cover (7) consists of pure cotton.

11. The nipple protector (1) according to one of claims 1 to 10, **characterized in that** the nipple protector (1) has an optical identification element (8) for identification of the nipple protector (1).

12. The nipple protector (1) according to claim 11, **characterized in that** the identification element is designed as a seam (8) of the cover (7) which differs visually from the remainder of the cover (7).

13. The nipple protector (1) according to claim 12, **characterized in that** the seam (8) differs in colour from the remainder of the cover (7).

14. The nipple protector (1) according to one of claims 1 to 13, **characterized in that** an active substance in the form of a liquid or ointment is accommodated in the base (2) and/or in the cover (7), which can be released to the breast (11) in the use position of the nipple protector (1).

15. Set comprising two nipple protectors (1) according to one of claims 11 to 14, wherein the two nipple protectors (1) can be distinguished visually from one another by means of the identification elements (7).

## Revendications

1. Dispositif de protection du mamelon (1) comprenant un corps de base (2) élastique et sensiblement annulaire avec une ouverture traversante (3) destinée à recevoir un mamelon (12) d'un sein (11) et de préférence une aréole (13) entourant le mamelon (12) dans la position d'utilisation du dispositif de protection du mamelon (1), **caractérisé en ce que** le dispositif de protection du mamelon (1) comprend en outre une housse (7) en textile qui est enfilée par-dessus le corps de base (2), l'ouverture traversante (3) n'étant pas couverte par la housse (7).

2. Dispositif de protection du mamelon (1) selon la revendication 1, **caractérisé en ce que** le dispositif de protection du mamelon (1) présente une hauteur (4) choisie de telle sorte que dans la position d'utilisation, le mamelon (12) ne dépasse pas au-delà de la face antérieure (9) du dispositif de protection du mamelon (1).

3. Dispositif de protection du mamelon (1) selon la revendication 2, **caractérisé en ce que** le corps de base (2) présente une section circulaire
ou **en ce que** le corps de base (2) présente une section avec une ligne de bord extérieure (15) elliptique
ou **en ce que** le corps de base (2) présente une section avec une ligne de bord extérieure (15) ovoïde,
l'aire de section étant dans chaque cas perpendiculaire à une direction dans laquelle la hauteur (4) est mesurée.

4. Dispositif de protection du mamelon (1) selon l'une des revendications 1 à 3, **caractérisé en ce que** le corps de base (2) a la forme d'un cylindre creux.

5. Dispositif de protection du mamelon (1) selon l'une des revendications 2 à 4, **caractérisé en ce que** la hauteur (4) du dispositif de protection du mamelon (1) varie sur une section du corps de base (2), l'aire de section étant perpendiculaire à une direction dans laquelle la hauteur (4) est mesurée.

6. Dispositif de protection du mamelon (1) selon la revendication 5, **caractérisé en ce que** le dispositif de protection du mamelon (1) est sensiblement en forme de coin.

7. Dispositif de protection du mamelon (1) selon l'une des revendications 1 à 6, **caractérisé en ce que** le corps de base (2) se compose d'une mousse, de préférence de mousse de polyester.

8. Dispositif de protection du mamelon (1) selon l'une des revendications 1 à 7, **caractérisé en ce que** le corps de base (2) est réalisé d'une pièce, de préférence par découpe à l'emporte-pièce.

9. Dispositif de protection du mamelon (1) selon l'une des revendications 1 à 8, **caractérisé en ce que** la housse (7) est réalisée en forme de tube et enfilée au moins une fois par-dessus le corps de base (2).

10. Dispositif de protection du mamelon (1) selon l'une des revendications 1 à 9, **caractérisé en ce que** le textile de la housse (7) est composé de pur coton.

11. Dispositif de protection du mamelon (1) selon l'une des revendications 1 à 10, **caractérisé en ce que** le dispositif de protection du mamelon (1) présente un élément d'identification visuelle (8) pour l'identification du dispositif de protection du mamelon (1).

12. Dispositif de protection du mamelon selon la revendication 11, **caractérisé en ce que** l'élément d'identification est réalisé comme une couture (8) de la housse (7) qui se détache visuellement du reste de la housse (7).

13. Dispositif de protection du mamelon (1) selon la revendication 12, **caractérisé en ce que** la couture (8) se détache du reste de la housse (7) par sa couleur.

14. Dispositif de protection du mamelon (1) selon l'une des revendications 1 à 13, **caractérisé en ce que** le corps de base (2) et/ou la housse (7) contiennent un principe actif sous forme de liquide ou de pommade, qui peut être libéré sur le sein (11) dans la position d'utilisation du dispositif de protection du mamelon (1).

15. Ensemble comprenant deux dispositifs de protection du mamelon (1) selon l'une des revendications 11 à 14, dans lequel les deux dispositifs de protection du mamelon (1) peuvent être distingués visuellement l'un de l'autre au moyen des éléments d'identification (7).
